# EUROPEAN PATENT APPLICATION

(11) **EP 4 609 847 A1**
(43) Date of publication of application: **03.09.2025**
(21) Application number: 24160762.1
(22) Date of filing: 01.03.2024
(51) Int. Cl.: A61K 8/04, A61K 8/34, A61K 8/35, A61K 8/36, A61K 8/37, A61K 8/60, A61K 8/67, A61K 8/97, A61Q 19/00, A61Q 19/04

(54) **ELECTROSTATICALLY SPRAYABLE TANNING FORMULATION**

(71) Applicant: IONIQ Skincare GmbH & Co. KG, 88677 Markdorf (DE)
(72) Inventor: Hajiesmaelian, Yashar, 81245 Munich (DE); Harnisch, Eva, 88214 Ravensburg (DE); Notheis, Manuel, 78351 Bodman-Ludwigshafen (DE)
(74) Representative: Kraus & Lederer PartGmbB

(57) **Abstract**

The present invention relates to an electrostatically sprayable topical formulation and production process thereof for giving skin a tanned appearance in the absence of ultraviolet radiation exposure and uniformly applied on the skin surface of a subject while maintaining its skin caring effect.

## Description

The present invention relates to an electrostatically sprayable topical formulation and production process thereof for giving skin a tanned appearance in the absence of ultraviolet radiation exposure and uniformly applied on the skin surface of a subject while maintaining its skin caring effect.

Usually, a tanned appearance is considered to be a healthy and attractive appearance. Therefore, many persons, especially younger persons, desire to a tanned appearance. Most of them obtain darker skin through exposure to UV light (e.g. sun-tanning or UV lamps). However, it is known that UV exposure results in accelerated skin aging and increase incidence of skin cancer.

Several artificial tanning products are known in the art that offer an alternative to natural or artificial sunlight. Some of them are described for example in US 2 949 403, EP 0 302 147 or EP 2 198 847. The products are also known as sunless tanning products. Sunless tanning products usually contain an active ingredient, such as reducing sugar or colorant, which causes the skin to assume browned appearance.

The sunless tanning products as known in the art are provided in form of an oil, lotion, or gel, whereby most of the products are lotions. Problems often associated with topical formulations such as tanning formulations include uneven application and streaking effect. If the amount of the tanning formulation, which is applied to the skin surface, is too much or too little, the area may become darker or lighter than the surrounding skin. As a result, the skin coloring may appear unnatural, streaked, or splotchy.

One reason for the uneven application and streaking effect of the tanning formulation is that most of the tanning formulations are applied on the surface of the skin by hand. However, this application method does not ensure that a sufficient amount of the tanning formulation is applied on the skin, in particular this application method does not provide a sufficient coverage of the skin and thus does not provide an even tanning appearance.

Another application method, which should ensure that a sufficient amount of the tanning formulation is applied on the surface of the skin is the use of pump sprays or pre-pressurized aerosol containers so as to have the formulation atomized and sprayed with the aid of propellant gas, as described for example in US 2005/0100516 or US 2003/0094510. However, conventional aerosol sprays frequently employ volatile compounds as propellants, which are environmentally unfriendly, possible hazardous to health and indeed are being legislated against in many countries. Moreover, it is hardly possible to generate a constant and homogenous flow of the tanning formulation. Therefore, also with this application method a uniform and sufficient coverage of the skin surface, i.e. a nearly 100% coverage, is not possible.

In the last few years in the field of cosmetic products a further application method has become of interest. In this application method the cosmetic topical formulation is applied on the subject by electrostatic spraying. Such a method is for example described in WO 94/11119, WO 2001/012139 or WO2021/052779.

This application method can be carried out without the aid of environmentally unfriendly propellants and provides a constant and uniform flow of the product. Hence, it is possible to apply a sufficient amount of the cosmetic formulation on the surface of the skin and thus a nearly full and uniform coverage of the skin may be possible.

In the prior art different electrostatic spraying devices are known, for example such devices are described in WO 94/11119, US 2010/0116897, DE 10 2017 108 610.2 or DE 102021109651. In an electrostatic spraying device, a voltage generator creates a high voltage which electrically charges the compounds of the cosmetic product. This results into a spray of the topical cosmetic formulation.

However, most of topical cosmetic formulations like tanning formulations are not electrostatically sprayable, because they do not have appropriate electrical characteristics, e.g. resistivity, permittivity etc., and/or have other properties such like a high surface tension, viscosity etc. which do not permit electrostatic spraying. For example, like many other cosmetic lotions and creams, tanning formulations include emollients, which are poorly electrostatic sprayable.

Emollients, also referred to as cosmetic oils, are compounds that soften and smooth the scales of the skin, which help to reduce rough and flaky skin. They are frequently occlusive agents, i.e. substances that provide a layer of protection that help prevent moisture (water) loss from the skin. Examples for emollients are silicones, such as dimethicone or cyclomethicone, vegetable oils, butters such as coca butter or shea butter, alcohols such as stearyl alcohol or cetyl alcohol, and petrolatum derivatives such a petroleum jelly or mineral oil. Due to their electrical characteristics, i.e., resistivity, conductivity, and permittivity, most of the emollients show a poor electrostatic sprayability.

Moreover, even though some of the tanning formulations have appropriate electrical characteristics, it is still not possible to electrostatically spraying these formulations such that all parts of the skin are covered by the formulation. This result also in a streaking effect on the skin surface, i.e. the applied topical formulation creates a "zebra crossing" pattern on the skin.

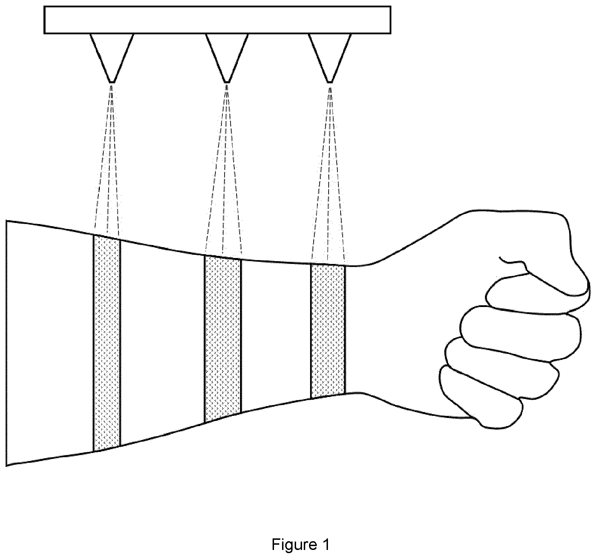

Figure 1 schematically shows the undesired "zebra crossing" pattern on the skin, which occurs if the topical cosmetic formulation is not uniformly sprayable and thus, uneven distribution of the formulation on the skin is possible.

Additionally, with respect to tanning formulations it has to be considered that most of the tanning formulations contain water to dissolve the water-soluble tanning agent(s) in the formulation. However, as for example described in US 2005/0100516, when water is atomized in an aerosol spray, the resulting particles have a tendency to be wet and course. This could cause the formulation to bead or clump on the skin and thus also resulting in an uneven or blotchy appearance. Hence, even though such tanning formulations may be electrostatically sprayable, the treated skin does not show the desired even tanning appearance.

It is known that the electrostatic spraying quality of the cosmetic formulation can be improved by raising the ethanol content of the formulation, see for example WO 2022/195069. However, a high concentration of ethanol in cosmetics or skin care products bears the risk of skin irritation, especially due to the loos of skin moisture. This undesired effect can be counteracted by using a sufficient quantity of emollients in the formulation, but, on the other hand, as mentioned above, the use of high amounts of emollients may worsen the electrostatic sprayability of the formulation.

Hence, object of the application was to provide an electrostatically sprayable topical formulation for giving skin a tanned appearance in the absence of ultraviolet radiation exposure, which can be easily applied and in a uniform manner on the skin surface of a subject to obtain a nearly full coverage of the skin surface and thus an even tanned appearance, while maintaining its skin caring effect.

It has been surprisingly found that this complex problem of the application can be solved by providing an electrostatically sprayable topical formulation comprising
a) less than 50 wt.-% of ethanol;
b) 20 to 55 wt.-% of water;
c) 0.5 to 5.0 wt.-% of at least one emollient, which is liquid at 20 °C and have a log K_{ow} of 2 to 10;
d) 0.05 to 2.0 wt.-% of at least one antioxidant, based on the total weight of the formulation;
e) 2 to 20 wt.-% of at least one tanning agent; and
f) 2 to 15 wt.-% of at least one cosmetic additive;
based on the total weight of the formulation.

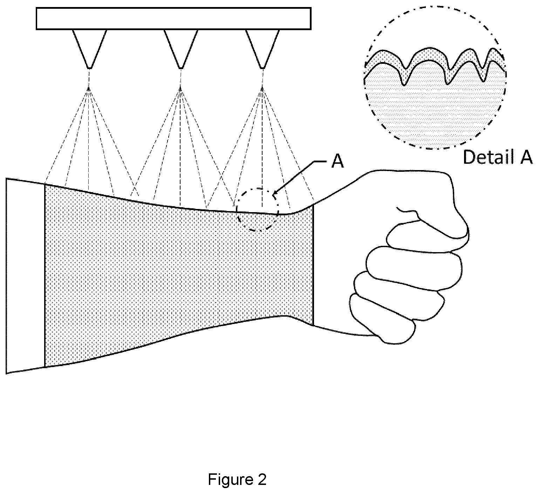

Figure 2 schematically shows the full coverage of the skin by using a topical formulation according to the invention, i.e. no streaking effect occurs. The enlarged section "Detail A" of Figure 2 shows a part of the skin, which is covered with the topical formulation of the invention.

For spaying the topical formulation on the skin surface of a subject, every electrostatic device suitable for cosmetic application as known in the art can be used. However, it is preferred to use a device as described in the examples below and which is commercially available under the name IONIQ ONE Sprayer. The use of a propellant for spraying the topical formulation of the invention on the skin surface of a subject is not necessary.

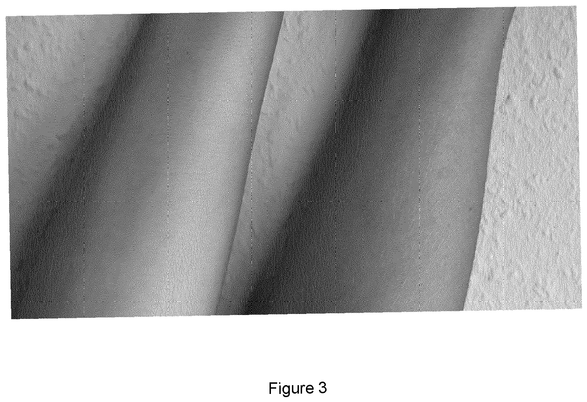

Figure 3 shows a right arm of a subject on which the topical formulation of the invention was applied by electrostatic spraying as described in the examples below in comparison to the untreated left arm of the subject. As can be seen from this picture the topical formulation of the invention provides an even and satisfying tanning appearance.

Furthermore, the topical formulation according to the invention provides at least a sufficient, in particular an improved, skin caring effect.

Before the sprayable topical formulation of the invention and production process thereof will be described in detail, it is to be understood that this invention is not limited to specific embodiments or conditions of the formulation described herein, since such embodiments and conditions may, of course, vary. It is also to be understood that the terminology used herein is not intended to be limiting, since the scope of the present invention will be limited only by the appended claims.

The abbreviation "wt.-%" or "w/w", as used herein, means "weight percentage" and refers to the weight amount of a compound in relation to the (total) weight of a formulation of compounds or of a substrate if nothing else is explicitly stated or obvious under the circumstances. Furthermore, the total sum of the weights of the components as described herein and present in the topical formulation of the invention is 100 %.

The recitation of numerical ranges by end points includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g., from 1.0 to 5.0 includes both 1.0 and 5.0). Any numerical range recited herein is intended to include all sub-ranges subsumed therein.

The term "comprising" includes "consisting essentially of" and also "consisting of".

In the present specification, the use of "a" in the singular also comprises the plural ("some"), and vice versa, unless the context clearly indicates the contrary.

The topical formulation of the invention can be used alone or in a mixture comprising other cosmetic formulations. Preferably, the topical formulation is a tanning formulation.

Even though the ethanol content of the topical formulation of the invention is less than 50 wt.-% to avoid any undesired skin irritation, the topical formulation of invention shows a satisfying electrostatic sprayability. Preferably, the formulation contains ethanol in amount of at least 25 wt.-%, or of at least 30 wt.-%, more preferably of at least 40 wt.-%, based on the total weight of formulation. According to the invention, it is preferred that the amount of ethanol present in the topical formulation is between 28.0 and 49.5 wt.-%, more preferably between 30 and 35 wt.-% or between 45.5 and 48.0 wt.-%, based on the total weight of the formulation.

Since the ethanol content present in the topical formulation of the invention is comparable low, the content of emollients, present in the topical formulation and which may influence the electrostatic sprayability of the formulation negatively, can be comparable low without losing the desired skin caring effect of the formulation.

According to the invention the topical formulation comprises at least one polar emollient that is liquid at 20 °C in amount of 0.5 to 5.0 wt.-%, preferably in an amount of 1.0 to 3.5 wt.-%, more preferably in an amount of 1.5 to 3.0 wt.-%, based on the total weight of the formulation.

In the cosmetic field, one possibility to define the polarity of a compound is to determine its n-octanol/water partition coefficient (K_{ow} or P). The partition-coefficient refers to the ratio of concentrations of the compounds in the mixture of these two immiscible phases at equilibrium. Hence, the partition coefficient measures how hydrophilic ("water-loving") or hydrophobic ("water-fearing") the tested compound is. In most of the cases the n-octanol/water partition coefficient is stated as decade logarithms log Kow or log P. The determination of the n-octanol/water partition coefficient is described for example in J. Sangster, "Octanol-Water Partition Coefficients: Fundamentals and Physical Chemistry", Vol. 2 of Wiley Series in solution chemistry, John Wiley & Sons, Chichester, 1997.

According to the invention, the log K_{ow} of the emollients, which are suitable for the topical formulation of the invention, is between 2.0 and 10.0, preferably between 2.5 and 9.5, 3.0 and 8.5, or between 3.5 and 7.5.

Examples for emollients which fulfill the above-mentioned requirements are known in the art. In particular, the emollients used in the topical formulation of the invention are preferably selected from the group consisting of dibutyl adipate, diisopropyl adipate, isononyl isononanoate, isopropyl palmitate, isodecyl neopentanoate, isoamyl laurate, ethyl macadamiate, octyldodecanol, PPG-15 stearyl ether, PPG-3 myristyl ether, PPG-14 butyl ether, dicaprylyl ether, dicaprylyl carbonate, isoamyl cocoate, diethylhexyl carbonate, isopropyl myristate, isopropyl palmitate, decyl cocoate, coco caprylate, coco caprylate/caprate, caprylyl caprylate/caprate, propylheptyl caprylate, phenoxyethyl caprylate, C12-C15 alkyl benzoate and combinations thereof.

More preferably the emollients are selected from the group consisting of dibutyl adipate, diisopropyl adipate, octyldodecanol, isodecyl neopentanoate, ethyl macadamiate, C12-C15 alkyl benzoate, and mixtures thereof. Most preferably, the emollients include at least dibutyl adipate.

If a mixture of emollients is used in the topical formulation of the invention, these emollients are used in different or equal amounts in the topical formulation of the invention so that the total amount of emollients is between 0.5 and 5.0 wt.-%, based on the total weight of the formulation.

It is state of the art that, as mentioned above, most of the tanning agents are water soluble, for example dihydroxyacetone (DHA). Therefore, it is mandatory that tanning formulations, and thus the topical formulation of the invention, contain a sufficient amount of water to ensure that the tanning agent is dissolved in the formulation. According to the teaching of the invention, the water content of the topical formulation is between 20 and 55 wt.-%, based on the total weight of the formulation. This ensures that the tanning agent is completely dissolved in the formulation, which contributes that the formulation can be uniform applied on the skin and thus a nearly full coverage of the skin can be achieved. Preferably, the water content of the inventive topical formulation is between 30 and 52 wt.-%, or between 31 and 50 wt.-%, more preferably between 40 and 48 wt.-%, based on the total weight of the formulation.

The invention relates to a topical formulation that provides a tanned appearance in the absence of ultraviolet radiation exposure. It is evident that the higher the amount of tanning agent present in the formulation the higher the tanning effect. The amount of tanning agent used in the invention results in a color change (tanning effect) of the treated skin noticeable by a subject. The color change can be determined by using an in vivo skin darkening test measuring the change in lightness as described for example in EP 2 198 848 or in PLoS ONE 15(12):e0233816, doi: 10.1371/journal.pone.0233816, "A colorimetric comparison of sunless with natural skin tan". The topical formulation of the invention comprises at least one tanning agent in an effective amount, i.e. in an amount of 2 to 20 wt.-%, based on the total weight of the formulation. According to the invention, the tanning agent is used preferably in an amount of 4 to 18 wt.-%, or 5 to 18 wt.-%, more preferably of 8 to 16 wt.-%, even more preferably of 10 to 14.0 wt.-%, or 11 to 12 wt.-%, based on the total weight of the formulation, in the topical formulation.

Self-tanning agents are generally selected from among mono- or polycarbonyl compounds. Suitable tanning agents for the topical formulation of the invention are isatin, alloxan, ninhydrin, glyceryl aldehydes, mesotartaric, aldehyde, glutaraldehyde, erythrulose, dihydroxyacetone (DHA), derivatives thereof, and combinations thereof. In particular it is preferred that the tanning agent is dihydroxyacetone (DHA), erythrulose or a mixture thereof. As for example disclosed in US 6 451 293 the combination of erythrulose with a reducing sugar, such as DHA, provides an even longer lasting tanned appearance. In case the reducing sugar is used in combination with erythrulose it preferred that the ratio of reducing sugar to erythrulose is 1:10 to 10:1, preferably 1:1, 3:1 or 2:1.

DHA is a 3-carbon sugar that when applied to the skin causes a chemical reaction with amino acids in the surface cells of the skin producing darkening effect. Other reducing sugars such as glucose, xylose, fructose, reose, ribose, arabinose, allose, tallose, altrose, mannose, galactose, sucrose, and lactose may be also suitable for the formulation of the invention. Preferably, according to the invention, DHA is used as tanning agent in the formulation.

One of the main problems which occurs with self-tanning agents is their instability, in particular during storage. In case DHA is used as tanning agent it is known that during storage the formulation release formaldehyde, which is harmful to health and produces a bad odor. For commercial use it is desirable that the tanning formulation of the invention is stable at room temperature for at least 6 months.

In order to ensure that the tanning agent is stable during storage, the topical formulation of the invention contains at least one antioxidant in an amount of 0.05 to 2.0 wt.-%, based on the total weight of the formulation. This amount ensures the stability of the tanning agent without negatively influencing the good electrostatic spraying quality of the formulation. It is preferred that the amount of the at least one antioxidant is between 0.1 and 1.0 wt.-%, more preferably between 0.15 and 0.8 wt.-% or 0.2 and 0.6 wt.-% based on the total weight of the formulation.

According to Römpp Chemie Lexikon, Stuttart/NewYork, Georg Thieme Verlag, 1995, antioxidants are compounds that inhibit or prevent undesired change in the substances to be protected caused by the action of oxygen, e.g. oxidative processes. Antioxidants are known in the prior art and for example described in Kosmetik International 2013 (8), pages 12-15. Examples for antioxidants suitable for cosmetic topical formulations are phenols, hydroquinones, pyrocatechols, sulphurous acid and salts thereof, aromatic compounds and amines, each of which may be substituted by sterically hindering groups, and metal complexes thereof.

Preferably, the topical formulation of the invention comprises at least one antioxidant selected from the group consisting of anoxomer, ascorbyl palmitate, butylated hydroxyanisole, butylated hydroxytolune, hypophosphorous acid, potassium metabisulfite, propyl octyl and dodecyl gallate, sodium metabilsulfite, sodium dioxide, tocopherols bis-ethyhexyl-hydroxydiemethodxy-benzylmalone, tocopherol, tocopheryl acetate, *rosmarinus officinalis* leaf extract, *humulus lupulus* extract, *salvia officinalis* leaf extract, and mixtures thereof.

It is more preferred that the antioxidant used in the formulation of the invention is bis-ethyhexyl-hydroxydiemethodxy-benzylmalone.

The topical formulation of the invention shows a stability of at least 6 months, more preferably of at least 12 months. In particular, the topical formulation shows a stability of 6 to 18 months, or preferably of 8 to 12 months.

Furthermore, the topical formulation comprises at least one cosmetic additive in an amount of 2 to 15 wt.-%, based on the total weight of the formulation. The cosmetic additives are preferably present in the topical formulation of the invention in an amount of between 2.5 wt.-% and 12.5 wt.-%, more preferably between 3 and 10 wt.-%, based on the total weight of the formulation. This ensures that the cosmetic additives used in the topical formulation of the invention do not influence negatively the electrostatic sprayability of the formulation, though provides the advantageous as known in the prior art due to the use of cosmetic additives.

In particular, it is preferred that the at least one cosmetic additive is a moisture agent or a mixture of moisture agents. Specific examples for moisture agents known in the art and suitable for the invention are fructose, glucose, maltitol, maltose, mannitol, glycerin, diglycerin, glyceryl glycoside, Xylitylglucoside, glycerol polymers, propylene glycol, pentylene glycol, propanediol 1,3 butylene glycol, hyaluronic acid, panthenol, inositol, betain, trehalose, xylitol, fructooligosaccharides, lactitol, linolenic acid, but also honey, hyaluronic acid, hydrogenated starch hydrolysate, natural moisturizing factor, acetylated lanolin and alcohol thereof, alanine, aspartic acid, barrier sphingolipids, ceresin, collagen, collagen, amino acids, serum protein, or extracts and oils of plants, like, beta vulgaris (Beet) root extract, algae extract, aloe barbadensis, aloe-barbadensis extract, aloe barbadensis gel, avocado (*persea gratissima*) oil, Calendula officinalis extract and oil thereof, althea officinalis extract, , arnica montana extract, birch (*betula alba*) bark extract, borage (*Borago officinalis*) extract, butcherbroom (*ruscus aculeatus*) extract, , evening primrose (*Oenothera biennis*) oil, nut oils for example, rice (*Oryza sativa*) bran oil, peppermint (*Mentha piperita*) oil, rosemary (*Rosmarinus officinalis*) oil, rose oil, safflower (*carthamus tinctorius*) oil, sage (*salvia officinalis*) oil, sandalwood (*santalum album*) oil, sesame (*Sesamum indicum*) oil, silk powder, wheat (*Triticum vulgare*) germ oil, and ylang ylang (*cananga odorata*) oil, etc.

In particular it is preferred that the topical formulation of the invention comprises at least one moisture agent selected from the group consisting of fructose, glucose, glycerin, glyceryl glycoside, propylene glycol, 1,3 butylene glycol, hyaluronic acid, panthenol, linolenic acid, hyaluronic acid, hydrogenated starch hydrolysate, acetylate lanolin alcohol, ceresin, collagen, collagen, algae extract, sweet almond oil, aloe barbadensis, aloe-barbadensis extract, aloe barbadensis gel, avocado oil, canola oil, *Calendula officinalis* extract, hydrogenated palm kernel oil, jojoba oil and mixtures thereof.

More preferably, the moisture agent is selected from the group consisting of glycerin, glyceryl glycoside, propylene glycol, panthenol, aloe barbadensis, aloe-barbadensis extract, aloe barbadensis gel, hyaluronic acid mixtures thereof. In a further preferred embodiment of the invention the moisture agent is at least glycerin, glyceryl glycoside, panthenol, propylene glycol and/or aloe-barbadensis extract.

The cosmetic additives used in the topical formulation of the invention may be further selected in addition to the moisture agents as described above from the group consisting of cosmetically acceptable carriers, oils, sterols, amino acids powders, colorants, pigments, dyes, pH adjusters, perfumes, essential oils, vitamins E, pro-vitamins, essential fatty acid, sphingolipids, UV filter etc. and mixtures thereof.

Preferably, according to the invention, the additives other than moisture agents are selected from the group consisting of perfumes, organic acids such as citric acid, vitamins and/or pro-vitamins, and combinations thereof.

In a specific embodiment of the invention, it is preferred that the topical formulation of the invention comprises at least one moisture agent in combination with at least one another cometic additive as defined above. In that case, it is further preferred that the at least one moisture agent is present in the topical formulation of the invention in an amount of 1.5 to 14.9 wt.-%, or 2.5 to 12.0 wt.-%, more preferably of 3.5 to 9.5 wt.-%, and the at least one other cosmetic additive in an amount of 0.1 to 5.0 wt.-%, or 0.5 to 3.0 wt.-%, more preferably of 0.8 to 2.5 wt.-%, based on the total weight of the formulation.

Furthermore, it is preferred that the topical formulation of the invention comprises citric acid. In particular, it is preferred that the citric acid is present in the formulation in an amount of 0.05 to 0.15 wt.-%, more preferably of 0.10 to 0.12 wt.-%, based on the total weight of the formulation. The citric acid is used in the preparation of the formulation for adjusting the pH of the formulation.

According to the teaching of the invention, it is preferred that the topical formulation has pH between 3.0 and 4.0, more preferably between 3.1 and 3.95.

Due to the good electrostatic sprayability of the topical formulation of the invention, there is no need that the formulation further comprises a propellant in order to ensure that the formulation is sprayable. In cosmetic and dermatology industry different propellants are used, for example, chemically-inert hydrocarbons such as propane, n-butane, isobutene and cyclopropane, and mixtures thereof, as well as halogenated hydrocarbons such as for example dichlorodifluormethane or 1,1-dichloro-1,1,2,2-tetrafluoroethane. isobutane, used singly or mixed with other hydrocarbons, particularly propane is for example preferred used in aerosol. The use of propellants involves numerous disadvantages that can be avoided by providing the formulation of the invention.

In a specific preferred embodiment of the invention, the electrostatic sprayable topical formulation according invention comprises / consists of
a) 40.0 to 48.5 wt.-%, preferably 47.8 wt.-%; of ethanol
b) 30.0 to 33.0 wt.-%, preferably 31 wt.-%, of water;
c) 2.0 to 4.0 wt.-%, preferably 3 wt.-%, of at least one emollient, which is liquid at 20 °C and have a log K_{ow} of 2 to 10;
d) 0.15 to 0.25 wt.-%, preferably 0.2 wt.-%, of at least one antioxidant;
e) 3.0 to 10.0 wt.-%, preferably 8 wt.-%, of at least one tanning agent; and
f) 2.5 to 12.5 wt.-% of cosmetic additives, based on the total weight of the formulation.

Preferably, the formulation of this specific embodiment comprises a combination of 2.0 to 10 wt.-% of at least one moisture agent and 0.5 to 2.5 wt.-%, of at least one cosmetic additive different from the at least one moisture agent, based on the total weight of the formulation, as cosmetic additives. Even more preferred is a combination of 9.2 wt.-% of at least one moisture agent and 0.7 wt.-% of another cosmetic additive, based on the total weight of the formulation. Furthermore, it is preferred that the at least one moisture agent is a mixture of moisture agents.

Additionally, the formulation of this specific embodiment preferably comprises 0.08 to 0.13 wt.-%, more preferably of 0.12 wt.-%, of citric acid, based on the total weight of the formulation.

In an alternative according to the invention, the electrostatic sprayable topical formulation of the invention comprises / consists of
a) 25 to 35 wt.-% of ethanol;
b) 35 to 55 wt.-% of water;
c) 0.5 to 2.5 wt.-% of at least one emollient, which is liquid at 20 °C and have a log K_{ow} of 2 to 10;
d) 0.15 to 0.25 wt.-% of at least one antioxidant;
e) 5.0 to 18 wt.-%, more preferably of 10 wt.-% to 16 wt.-%, of at least one tanning agent; and
f) 2.5 to 12.5 wt.-% of cosmetic additives, based on the total weight of the formulation.

Preferably, the formulation of this specific embodiment comprises a combination of 2.0 to 10 wt.-% of at least one moisture agent and 0.5 to 2.5 wt.-%, of at least one cosmetic additive different from the at least one moisture agent, based on the total weight of the formulation, as cosmetic additives. Furthermore, it is preferred that the at least one moisture agent is a mixture of moisture agents.

The electrostatic sprayable topical formulation of the invention can be obtained by any method usually used for the preparation of cosmetic formulations. However, preferably, the electrostatic sprayable topical formulation of the invention is obtained by a process comprising the following process steps:
(i) adding the tanning agent to water;
(ii) stirring the mixture of tanning agent and water until the mixture is homogenous and clear;
(iii) adding the at least one cosmetic additive, if the at least one cosmetic additive is a moisture agent or mixture thereof, to the mixture obtained in step (ii) and stirring the resulted mixture until the mixture is homogenous and clear;
(iv) optionally adjusting the pH of the mixture obtained in step (ii) or (iii) to a pH of between 1.5 and 4, preferably between 2 and 3;
(v) adding ethanol to the mixture obtained in step (ii), (iii) or (iv) and stirring the resulted mixture until the mixture is homogenous and clear; and
(vi) adding at least one emollient, at least one antioxidant, and optionally at least one cosmetic additive, which differs from the moisture agent of step (iii), to the mixture obtained in step (v) and stirring the resulted mixture until the mixture is homogenous and clear to obtain the topical formulation of the invention.

The production process of the invention is conducted preferably at room temperature. It is further preferred that in step (iv) the pH of the mixture obtained in step (ii) or (iii) is adjusted to the desired pH by using citric acid. The citric acid used in this process step is preferably in powder form, i.e. water free.

The produced electrostatic sprayable topical formulation, i.e. the formulation of the invention comprising the specific combination of components as described above, is a clear liquid and has an electrostatic conductivity of 0 to 45 µS/cm, more preferably of 1 to 40 µS/cm, even more preferably of 5 to 30 µS/cm, measured at 21 °C and by the method as described in the examples.

Furthermore, the topical formulation of the invention preferably has a density of 0.920 to 0.970 g/ml, more preferably of 0.925 to 0.965 g/ml at room temperature.

The viscosity of the topical formulation of the invention is preferably in the range of 1.0 to 200 mPas, more preferably of 2.0 to 180 mPas, even more preferably of 5.0 to 150 mPas, measured by the method as described in the examples.

The example that follows is intended for illustrating the invention in more detail.

### Examples

### Measurement methods

### 1. Electrical conductivity

The electrical conductivity was measured by means of PCE Instruments PCE-PH 30. Measurement at 21 °C temperature.

### 2. Viscosity

The viscosity was determined by means of IKA Viskosit6tsmessger6t ROTAVISC lo-vi. The measurement was performed at room temperature by Sp01-60 rpm for 60 seconds.

### 3. Spraying test

The topical formulations as described below was sprayed by using an IONIQ ONE Sprayer, which is commercially available since July 01, 2021 from IONIQ skincare GmbH & Co. KG.

The sprayer has the following characterizations:

| | |
|---|---|
| Voltage: | 8 kV |
| Diameter of spraying nozzle: | approx. 90 µm |
| Number of the nozzles: | 3 |
| Feed rate: | 8 ml/min (total) |

In order to determine whether the topical formulation provides a homogenous distribution and a sufficient tanned appearance on the skin of a subject, the topical formulation was applied on the skin of the subject in the following manner:
The tested cosmetic formulation was slowly sprayed with a distance of 25 cm on the arm of the test person. The spraying time was approx. 15 second. The formulation was leak to soak for 10 minutes. After 8 hours the subject took a shower and assesses the spraying result.

### Example

### 1. Composition of the formulation:

**Table 1**

| **Material** | **INCI** | **amount** [wt.-%] | **Phase** |
|---|---|---|---|
| **Water,** demin. | AQUA | 31.00 | A1 |
| **Tanning Agent:** | DIHYDROXYACETONE | 8.00 | |
| Dihydroxyaceton (DHA), extra pure | | | |

| **Moisture Agents:** | | | |
|---|---|---|---|
| Propylenglycol Ph. Eur. 7.0 | PROPYLENE GLYCOL | 5.00 | A2 |
| Panthenol, D-75% | PANTHENOL | 0.20 | |
| Hydagen Aquaporin | GLYCERYL GLYCOSIDE | 2.00 | |
| Aleo Extract in aqueous glycerin | ALEO EXTRACT | 1.00 | |
| Glycerin 99.5% | GLYCERIN | 1.00 | |
| **Total amount of moisture agents:** | | 9.20 | |
| Citric acid (100%) | CITRIC ACID | 0.12 | B |
| **Ethanol** (98%) | ALCOHOL DENAT. | 47.78 | C |
| **Emollient:** | DIBUTYL ADIPATE | 3.00 | D |
| Cetiol B | | | |
| **Antioxidant:** | BIS-ETHYHEXYL | 0.20 | |
| RonaCare AP^{™} | HYDROXYMETHOXY | | |
| | BENZYLMALONATE | | |
| **Additive:** | PERFUME | 0.7 | |
| Perfume | | | |

### 2. Production Process:

For the production of the formulation a sufficiently large vessel equipped with a stirrer was used.

The amounts of components indicated as wt.-% in the above Table 1 correspond to the grams of the components used in the production process to obtain a topical formulation according to the invention.

The production process was conducted at room temperature in the following manner:
(i) Water was submitted into the vessel and subsequently DHA as tanning agent was added.
(ii) Afterwards the resulted mixture was stirred to obtain a homogenous and clear solution indicated as phase A1 (see Table 1 above).
(iii) The compounds of phase A2 were added to phase A1 and the obtained mixture of phases A1 and A2 was stirred until the mixture was again homogenous and clear.
(iv) The pH of the mixture of phases A1 and A2 was adjusted to 2-3 by adding citric acid (phase B) to the mixture.
(v) Subsequently, ethanol (phase C) was added and the resulted mixture (phases A1 + A2 + B + C) was stirred until a homogenous and clear mixture was obtained.
(vi) Finally, the compounds of phase D were added to the mixture and stirred until a homogenous and clear solution of the topical formulation according to the invention was obtained.

### 3. Results:

The produced formulation was liquid, had a density of 0.947 g/ml at 21 °C and a pH of 3.55. The electric conductivity of the formulation was 26 µm/cm at 21 °C measured by the method as described above.

In Figure 3 it is demonstrated that the such produced formulation could be uniformly applied on the skin of a subject by electrostatic spraying as described above and provides a satisfying tanning appearance.

## Claims

**1.** A electrostatically sprayable topical formulation comprising
a) less than 50 wt.-% of ethanol;
b) 20 to 55 wt.-% of water;
c) 0.5 to 5.0 wt.-% of at least one emollient, which is liquid at 20 °C and have a log K_{ow} of 2 to 10;
d) 0.05 to 2.0 wt.-% of at least one antioxidant, based on the total weight of the formulation;
e) 2 to 20 wt.-% of at least one tanning agent; and
f) 2 to 15 wt.-% of at least one cosmetic additive;
based on the total weight of the formulation.

**2.** The electrostatically sprayable topical formulation according to claim 1, wherein the at least one cosmetic additive is selected from the group consisting of moisture agents, organic acids, perfumes, vitamin, pro-vitamins and mixtures thereof.

**3.** The electrostatically sprayable topical formulation according to claim 1 or 2, wherein the cosmetic additive is at least one moisture agent or mixtures thereof.

**4.** The electrostatically sprayable topical formulation according to any one of the preceding claims, wherein the formulation comprises at least 25 wt.-% of ethanol, based on the total weight of the formulation.

**5.** The electrostatically sprayable topical formulation according to any one of the previous claims, wherein the tanning agent is dihydroxyacetone.

**6.** The electrostatically sprayable topical formulation according to any one of the previous claims, wherein the at least one emollient is selected from the group consisting of dibutyl adipate, diisopropyl adipate, isononyl isononanoate, isopropyl palmitate, isodecyl neopentanoate, isoamyl laurate, ethyl macadamiate, octyldodecanol, PPG-15 stearyl ether, PPG-3 myristyl ether, PPG-14 butyl ether, dicaprylyl ether, dicaprylyl carbonate, isoamyl cocoate, diethylhexyl carbonate, isopropyl myristate, isopropyl palmitate, decyl cocoate, coco caprylate, coco caprylate/caprate, caprylyl caprylate/caprate, propylheptyl caprylate, phenoxyethyl caprylate, C12-C15 alkyl benzoate and combinations thereof.

**7.** The electrostatic sprayable topical formulation according to any one of the preceding claims, wherein the antioxidant is bis-ethyhexyl-hydroxydimethoxy-benzylmalonate.

**8.** The electrostatically sprayable topical formulation according to any one of claims 2 to 7, wherein the moisture agent is selected from the group consisting of glycerin, propylene glycol, aloe-barbadensis extract, panthenol, glyceryl glycoside and mixtures thereof.

**9.** The electrostatically sprayable topical formulation according to any one of the preceding claims, wherein the formulation comprises citric acid.

**10.** The electrostatically sprayable topical formulation according to any one of the preceding claims, wherein the formulation does not comprise a propellant.

**11.** The electrostatic sprayable topical formulation according to any one of the preceding claims, wherein the formulation comprises
a) 40.0 to 48.5 wt.-% of ethanol;
b) 30.0 to 33.0 wt.-% of water;
c) 2.0 to 4.0 wt.-% of at least one emollient, which is liquid at 20 °C and have a log K_{ow} of 2 to 10;
d) 0.15 to 0.25 wt.-% of at least one antioxidant;
e) 3.0 to 10.0 wt.-% of at least one tanning agent; and
f) 2.5 to 12.5 wt.-% of cosmetic additives, based on the total weight of the formulation.

**13.** The electrostatic sprayable topical formulation according to any one of claims 1 to 11, wherein the topical formulation comprises
a) 25 to 35 wt.-% of ethanol;
b) 35 to 55 wt.-% of water;
c) 0.5 to 2.5 wt.-% of at least one emollient, which is liquid at 20 °C and have a log K_{ow} of 2 to 10;
d) 0.15 to 0.25 wt.-% of at least one antioxidant;
e) 5.0 to 18 wt.-% of at least one tanning agent; and
f) 2.5 to 12.5 wt.-% of cosmetic additives, based on the total weight of the formulation.

**14.** A process for the preparation of the electrostatic sprayable topical formulation according to any one of claims 1 to 13, wherein process comprises the following steps:
(i) adding the tanning agent to water;
(ii) stirring the mixture of tanning agent and water until the mixture is homogenous and clear;
(iii) adding the at least one cosmetic additive, if the at least one cosmetic additive is a moisture agent or mixture thereof, to the mixture obtained in step (ii) and stirring the resulted mixture until the mixture is homogenous and clear;
(iv) optionally adjusting the pH of the mixture obtained in step (ii) or (iii) to a pH of between 1.5 and 4, preferably between 2 and 3;
(v) adding ethanol to the mixture obtained in step (ii), (iii) or (iv) and stirring the resulted mixture until the mixture is homogenous and clear; and
(vi) adding at least one emollient, at least one antioxidant, and optionally at least one cosmetic additive, which differs from the moisture agent of step (iii), to the mixture obtained in step (v) and stirring the resulted mixture until the mixture is homogenous and clear.

**15.** Use of an electrostatic sprayable topical formulation according to any one of claims 1 to 13, wherein the topical formulation is topically applied to the skin of a subject by electrostatic spraying.
